# EUROPEAN PATENT APPLICATION

(11) **EP 4 563 543 A1**
(43) Date of publication of application: **04.06.2025**
(21) Application number: 23213401.5
(22) Date of filing: 30.11.2023
(51) Int. Cl.: C03C 17/00, A61J 1/05, A61J 1/14, A61M 5/28, C03C 17/30

(54) **METHOD AND GLASS CONTAINER FOR STORING A PHARMACEUTICAL COMPOSITION AT A TEMPERATURE OF -80 °C OR BELOW**

(71) Applicant: SCHOTT Pharma Schweiz AG, 9001 St. Gallen (CH)
(72) Inventor: HEIZ, Benjamin, 9001 St.Gallen (CH); BABIC, Ugljesa, 9001 St. Gallen (CH); GONCALVES, Elsa Silva, 9001 St. Gallen (CH); VLADISLAVOVA, Liliya, 9001 St. Gallen (CH); HAHN, Christian, 9403 Goldbach (CH); HEINECKE, Tobias, 9001 St. Gallen (CH); STUMPF, Sören, 9001 St. Gallen (CH)
(74) Representative: Schott Corporate IP

(57) **Abstract**

This disclosure relates to a method for storing a pharmaceutical composition at a temperature of -80 °C or below, which comprises defined cooling rates, and a glass container suitable for this purpose, which comprises a coating whose crystallization temperature range and melting temperature range is overlapping.

## Description

The present disclosure relates to a method for storing a pharmaceutical composition at a temperature of -80 °C or below, which comprises defined cooling rates, and a glass container suitable for this purpose, which glass container comprises a coating whose crystallization temperature range and melting temperature range are overlapping.

### Background

Glass containers for storage and delivery of pharmaceutical compositions are as such known in the prior art. Such containers usually feature a stopper useful for eluting the contents of the container through an outlet and a closure. The stopper must slide within the container and yet provide for a tight seal so that the composition remains safely stored even for extended time periods.

However, if such a pharmaceutical container prefilled with a pharmaceutical composition is stored at low temperatures, there is a risk that the tight seal is compromised either during cooling or thawing or during storage and transport. Many drugs, such as mRNA vaccines, require a temperature of -80 °C or below for safe storage and transport which is usually achieved by using dry ice whose sublimation temperature is -80 °C. During transport, the temperatures may punctually reach -96 °C. Hence, long-term container closure integrity (CCI) must be ensured at -80 °C and shorter periods at -96 °C.

It is, therefore, an object of the present invention to provide glass containers that overcome the problems of the prior art and that can be used for storing pharmaceutical compositions at temperatures of -80 °C or below. A further object of the invention is to provide a method for storing pharmaceutical compositions.

### Summary of disclosure

In a first aspect, the present disclosure relates to a method for storing a pharmaceutical composition at a temperature of -80 °C or below comprising
- providing a glass container comprising a hollow cylindrical body having at least one open end and at least one stopper closing the at least one open end, wherein at least a part of an inner surface of the glass container comprises a coating, the coating having a crystallization temperature range and a melting temperature range determined using differential scanning calorimetry at a temperature change rate of 10 °C/min, wherein the crystallization temperature range and the melting temperature range overlap at a temperature from -75 °C to -100 °C, particularly at -80 °C;
- filling a pharmaceutical composition into the glass container; and
- cooling the glass container with a cooling rate of ≤ 6.0 °C/min in a range of from -50 °C to -80 °C and with a cooling rate of ≤ 5.0 °C/min in a range from -80 °C to -96 °C.

In a second aspect, the present disclosure relates to a glass container for storing a pharmaceutical composition at a temperature of -80 °C or below, comprising a hollow cylindrical body having at least one open end and at least one stopper closing the at least one open end, wherein at least a part of an inner surface of the glass container comprises a coating, the coating having a crystallization temperature range and a melting temperature range determined using differential scanning calorimetry at a temperature change rate of 10 °C/min, wherein
- the crystallization temperature range and the melting temperature range overlap at a temperature from -75 °C to -100 °C, particularly at -80 °C, and
- the coating has a thickness of 400.0 nm or more or of 800.0 nm or more, and/or of 2,000.0 nm or less or of 1,500.0 nm or less, and
- when cooling the glass container with a cooling rate of ≤ 6.0 °C/min in a range from -50 °C to -80 °C and with a cooling rate of ≤ 5.0 °C/min in a range of from -80 °C to -96 °C, at a temperature between the glass transition temperature of the coating and the storing temperature a distance between an outer surface of the stopper and the inner surface of the glass container which comprises the coating changes not more than 1.0 %.

Here, the distance is referring to the distance between the glass surface without the applied coating and the surface of the stopper. It is, hence, equivalent to the space occupied by the coating when the sealing is intact, i.e., when there are no voids. Moreover, the change of this distance is also a measure for the stress applied to the coating during the cooling procedure.

### Brief description of the figures

- **Figure 1**: Glass container with a coating.
- **Figure 2**: Illustration of ethanol-modified dye ingress container closure integrity test.
- **Figure 3**: DSC diagram of a coating according to this disclosure.
- **Figure 4**: DSC diagram of a coating according to this disclosure.
- **Figure 5**: DSC diagram of a prior art coating.
- **Figure 6**: DSC diagram of a prior art coating.
- **Figure 7**: Diagram of a TMA analysis on a coating according to this disclosure.
- **Figure 8**: Diagram of a TMA analysis on a coating according to this disclosure.

### Details of disclosure

Details of this disclosure relate to the aspects described in the summary of this disclosure. Any of the features of the embodiments described hereinafter may relate to the method for storing a pharmaceutical composition at a temperature of -80 °C or below as well as the glass container used for that purpose.

Throughout this application, room temperature is preferably a temperature of 293.15 K, especially at 1013.25 hPa.

The glass container may be any type of container, including a vial, a syringe, or a cartridge.

In an embodiment, the glass container is a pre-filled syringe or cartridge. This is particularly advantageous because this way the pharmaceutical composition can be stored directly in the syringe or cartridge at low temperatures. This makes it very easy to handle the composition because the syringe or cartridge is prefilled and can be used for administration without transferring the composition to a different container. All the more, this is even possible if the storage temperature of the composition is below a temperature where an expansion of the composition takes place.

### Exemplary container

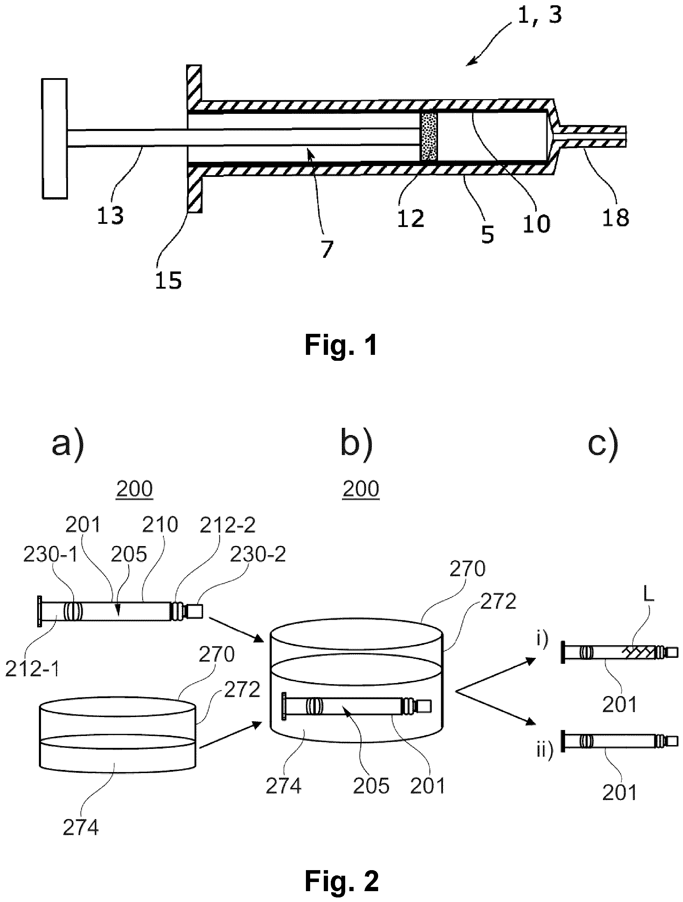

Referring to the drawings, **Figure 1** illustrates a glass container 1, in an exemplary embodiment a syringe 3, for administering pharmaceuticals or cosmetics. The syringe 3 is made of glass and comprises a glass wall 5 surrounding a lumen. The container comprises a hollow cylindrical body 7 and a nestling surface 18 onto which, for example, an injection needle or a cap can be placed. A stopper 12 is inserted in the cylindrical portion and is slidable in the axial direction by pressure on a push rod 13. The cylindrical portion has a flange 15 for handling purposes at the end of the introduction opening for the stopper 12.

The glass container 1 is provided with a coating 10 on an inner surface, here specifically on the inner surface of the hollow cylindrical body 7. In this example, the coating 10 covers that region of the inner surface of the hollow cylindrical body 7 over which the stopper 12 can slide when the syringe is being emptied or used for drawing up.

This disclosure is not particularly limited to container volumes. In an embodiment, the hollow cylindrical body encloses a volume of at least 0.10 ml, at least 0.50 ml, or at least 1.00 ml. Optionally, the volume may be up to 1,000 ml, up to 200 ml, up to 100 ml, or up to 25 ml. In embodiment, the volume ranges from 0.1 ml to 1,000 ml, from 0.50 ml to 200 ml, or from 1.00 ml to 25 ml. In an embodiment, the hollow cylindrical body encloses a volume of less than 10.0 ml.

The hollow cylindrical body has a lumen surrounded by a glass wall, wherein the glass wall may have a wall thickness of at least 0.50 mm, at least 0.80 mm, or at least 1.00 mm. Optionally, the glass wall thickness may range up to 10.0 mm, up to 8.0 mm, up to 5.0 mm, or up to 4.0 mm. In embodiments, glass wall thickness is from 0.50 mm to 10.0 mm, from 0.80 mm to 8.0 mm, or from 1.00 mm to 4.00 mm. The term "wall thickness" as used herein describes the shortest distance between an inner surface and an outer surface of the hollow cylindrical body.

The term "outer diameter" as used herein refers to the maximum distance between two points on the outer surface of the hollow cylindrical body, wherein the two points are connected by a straight line, which is perpendicular to and intersects with the longitudinal axis of the hollow cylindrical body. The term "inner diameter" as used herein refers to the maximum distance between two points on the inner surface of the hollow cylindrical body, wherein the two points are connected by a straight line, which is perpendicular to and intersects with the longitudinal axis of the hollow cylindrical body.

The hollow cylindrical body of the container may have an essentially constant inner diameter. This means that a total inner diameter variation is low. The "total inner diameter variation" is the difference between the largest inner diameter of a hollow cylindrical body and the smallest inner diameter of the same hollow cylindrical body. For example, a total inner diameter variation of a hollow cylindrical body may be less than 200 µm, less than 100 µm, less than 50 µm, or less than 25 µm. Optionally, a total inner diameter variation may be 0.01 µm or more, 0.10 µm or more, or 1.0 µm or more.

### Cooling procedure

The inventors have found that a key element for achieving CCI of glass containers at -80 °C or below is the cooling procedure and container configuration adapted in this regard. While certain siloxane-based coatings have proven themselves to be suitable for achieving CCI at temperatures of -60 °C and below, they have faced CCI problems at temperatures of -80 °C and below, in particular at -96 °C. Further investigation of the sealing between a stopper and a glass container by the coating has revealed that during the cooling procedure the action of different forces on the coating layer which is losing its elasticity at such low temperatures causes the defects in the coating. The differing thermal expansion or contraction behavior of the various components of the system, i.e., the rubber of the stopper, the glass of the container, the coating components, and the pharmaceutical composition, create axial and radial forces on the coating which when unbalanced will fracture the embrittled coating. The elasticity of the stopper and/or coating governs the container closure integrity of the container under dynamic conditions. Once the glass transition temperatures of both materials are crossed, the system shall be as close as possible to its equilibrium, i.e., no further force should act on the stopper/coating interface. If the stopper moves, the embrittled coating gets damaged and CCI is consequently not maintained anymore.

The solution to this problem has been found to be a specific cooling procedure with a cooling rate of ≤ 6.0 °C/min in a range of from -50 °C to -80 °C and with a cooling rate of ≤ 5.0 °C/min in a range of from -80 °C to -96 °C. Above -50 °C, the cooling rate may be set to higher rates but it has to be made certain that the temperature will decrease with a rate ≤ 6.0 °C/min when passing -50 °C. That is to say that an "overshooting" of the temperature has to be avoided by reducing the cooling rate in time. Preferably, the cooling rate of ≤ 6.0 °C/min is applied in the whole range of room temperature, i.e., 20 °C, to -80 °C. With these rates, an optimum balance between the shrinking and relaxing rubber of the stopper, the shrinking of the coating and the glass container, and the expansion of the filling medium can be achieved. The coating used in the procedure must have a crystallization temperature range and melting temperature range which overlap at a temperature of from -75 °C to -100 °C, particularly at -80 °C. On the one hand, such a coating is able to maintain CCI at the desired temperatures when cooled with these cooling rates. And on the other hand, the cooling procedure is tailored with the applicable temperature ranges to these properties of the coating.

Thus, the inventors have overcome the prevailing prejudice in the prior art that glass containers, in particular in the form of prefilled syringes, with silicone oil as gliding agent could not maintain CCI at temperatures below about -60 °C. This prejudice is attributed to the fact that the silicone oil reaches its glass transition temperature at about -60 °C, and further to the known difficulty with glass containers as opposed to polymer containers having a larger difference in the coefficient of thermal expansion between the container material and the material of the stopper which is usually a rubber material. The lower the temperatures become, the more stress is generated at the sealing surfaces.

In embodiments, cooling the glass container may comprise keeping the temperature constant for at least 1.0 min, or at least 2.0 min, or at least 3.0 min, or at least 4.0 min, or at least 5.0 min in a range of 1.0 °C to 5.0 °C each above the glass transition temperature of the stopper and the coating.

By introducing these temperature plateaus in the cooling procedure slightly above the especially critical glass transition temperatures, the system is allowed to equilibrate before crossing them. This may help to further reduce the forces occurring on the coating.

### Coating

The coating may be disposed on an inner surface of the hollow cylindrical body of the glass container and/or on one or more other surfaces of the glass container, including surfaces at the tip of a syringe, such as the nestling surface at the tip side of a syringe. At least a part of an inner surface of the glass container comprises the coating. The coatings and coating compositions described herein help achieve a tight seal at low temperatures.

The coating may be amorphous, or partially crystalline at 20 °C. Optionally, the coating has a crystallinity of less than 20 % (v/v) at 20 °C.

In embodiments, the coating may have a glass transition temperature at -60 °C or below, or at -70 °C or below, or at -75 °C or below, or at -80 °C or below; and/or the stopper may have a glass transition temperature at -80 °C or below, or at -85 °C or below, or at -90 °C or below, or at -95 °C or below, or at -100 °C or below, or below the storing temperature.

Optionally, the glass transition temperature of the coating may be at -200 °C or higher, at -150 °C or higher, or at -120 °C or higher, or at -100 °C or higher. Glass transition temperature may be measured using differential scanning calorimetry (DSC), or thermomechanical analysis (TMA). An exemplary way of determining a coating's glass transition temperature includes thermomechanical analysis in expansion mode, for example using a Q400 thermomechanical analyzer by TA Instruments. The sample may be prepared by coating a glass container according to this disclosure, scraping off the coating using a scalpel and performing thermomechanical analysis in expansion mode, i.e., measuring the sample's expansion or contraction as a function of temperature. In embodiments, a glass transition temperature of the coating is in a range from -200 °C to -60 °C, from -150 °C to -70 °C, from -120 °C to -75 °C, or from -100 °C to -80 °C. In one embodiment, the glass transition temperature is from -90 °C to -80 °C.

In embodiments of this disclosure, the coating has a crystallization temperature range and a melting temperature range determined using differential scanning calorimetry at temperature change rate of 10 °C/min, wherein the crystallization temperature range and the melting temperature range overlap at a temperature of from -75 °C to -100 °C, particularly at -80 °C. For example, DSC may be performed in a temperature range of -120 °C to -60 °C. A suitable instrument is a DSC Q2000 (TA Instruments).

Without wishing to be bound by this theory, the inventors hypothesize that, within the range of overlap, both crystalline and molten portions of the coating exist. This is believed to give the coating the properties in terms of mechanical resistance and elasticity needed to form a tight seal at low temperatures. Crystallization temperature range and melting temperature range are considered to overlap, if the crystallization and melting peak areas extend into the same temperature range. For example, crystallization may start at -55 °C and end at -95 °C, i.e., an exothermal crystallization peak area may range from -55 °C to -95 °C; melting may start at -90 °C and end at -40 °C, i.e., an endothermal melting peak area may range from -90 °C to -40 °C. In this example, the temperature range of overlap is -90 °C to -55 °C. This example fulfills the requirement of an overlap at a temperature from -75 °C to -100 °C because there is an overlap at at least one temperature within the indicated range.

In embodiments, the coating may have a thickness of 400.0 nm or more or of 800.0 nm or more, and/or of 2,000.0 nm or less or of 1,500.0 nm or less. In some embodiments, the coating may have a thickness of 400.0 nm or more, or 500.0 nm or more, 600.0 nm or more, or 700.0 nm or more, 800.0 nm or more, or 900.0 nm or more, or 1,000.0 nm or more. In some embodiments, the coating may have a thickness of 2,000.0 nm or less, or 1,900.0 nm or less, or 1,800.0 nm or less, or 1,700.0 nm or less, or 1,600.0 nm or less, or 1,500.0 nm or less, or 1,400.0 nm or less. Optionally, the coating may have a thickness in the range of from 400.0 nm to 2,000.0 nm, or of from 500.0 nm to 1,900.0 nm, or of from 600.0 nm to 1,800.0 nm, or of from 700.0 nm to 1,700.0 nm, or of from 800.0 nm to 1,600.0 nm, or of from 900.0 nm to 1,500.0 nm, or of from 1000.0 nm to 1,400.0 nm.

The inventors have found that a certain minimum thickness of the coating is preferable for achieving CCI at -80 °C or below. The thickness has to be sufficient for sealing the contact surface between the glass container and the stopper during the freezing and thawing procedure as well as during the transport and storage. To this end, there has to be enough material present to fill the gap between the glass wall and the stopper which is increasing with decreasing temperature due to the different coefficients of thermal expansion of the respective glass and rubber materials. However, if the coating is too thick, it may cause difficulties when the elasticity decreases in the course of decreasing temperature and shear forces occur on the sealing area due to a contracting or expanding filling of the container and/or a plunger movement. Hence, the thickness of the coating should be optimized for the respective container-coating-stopper configuration for extending times and temperature for storage and transport to the longest and deepest values, respectively.

The coating may contain one or more silicon-organic polymers. A "silicon-organic polymer" is a polymeric material comprised of monomeric units, the monomeric units comprising both silicon (Si) and carbon (C) atoms. An example of a silicon-organic polymer is a polysiloxane. In embodiments, the coating comprises one or more polysiloxane structural units. "Polysiloxane structural units" may refer to polysiloxane structures within a larger molecule (e.g., covalently bonded to a larger molecule, or part of a larger molecule) or to polysiloxane molecules per se. For example, cross-linked polysiloxane structural units are part of (covalently attached to) a polymeric network, whereas non-cross-linked polysiloxane structural units are present in the coating as molecules without being covalently attached to other molecules in the coating. Thus, the coating may comprise cross-linked polysiloxane structural units and/or non-cross-linked polysiloxane structural units. In this context, "cross-linked" means that the polysiloxane structural units are covalently attached to a polymeric network. Specifically, the term "cross-linked" includes the preferred case that a polysiloxane structure is covalently linked to other polysiloxane structures, e.g., through a polymeric backbone. Optionally, the cross-linked polysiloxane structural units are covalently bonded to other polysiloxane structures as a result of a hydrosilylation reaction. The polymeric backbone may for example be formed by polymerizing a polysiloxane carrying a polymerizable functional group such as a vinyl group. In contrast, "non-cross-linked" means that the polysiloxane is not covalently linked to other polysiloxane structures through a polymeric backbone, or preferably not covalently linked to other polysiloxanes of the coating at all.

In an embodiment, the cross-linked polysiloxane structural units are cross-linked via one or more, e.g., two, end groups. The end groups may be selected from vinyl, acryl, methacryl, styrene, and combinations thereof. In an embodiment, the coating comprises a hydrosilylation reaction product of a cross-linkable polysiloxane compound and a cross-linking polysiloxane compound, such as a vinyl-polysiloxane compound and a polysiloxane having at least two Si-H groups. The cross-linking polysiloxane may cross-link the cross-linkable polysiloxane by reaction of its plurality of Si-H groups with vinyl groups of the cross-linkable polysiloxane. The reaction may be catalyzed by metals such as Pt, Pd, Cu, Ti, or V. Preferably, the reaction may be platinum catalyzed.

In this disclosure, "polysiloxane" or "polysiloxane structural unit" may refer to polyalkylsiloxane structural units, such as polydialkylsiloxane structural units. Optionally, one or more of the alkyl groups in the polyalkylsiloxane or polydialkylsiloxane are independently selected from branched or unbranched C, to C₈ alkyl groups. The alkyl groups may be linear alkyl groups. For example, the alkyl groups may be independently selected from methyl, ethyl, propyl, butyl, pentyl, hexyl, heptyl and octyl groups. Preferably, the alkyl groups are independently selected from methyl and ethyl.

In embodiments, the coating may comprise one or more cross-linked polysiloxane structural units and one or more non-cross-linked polysiloxane structural units, wherein a ratio of a weight amount of cross-linked polysiloxane structural units to a weight amount of non-cross-linked polysiloxane structural units in the coating is less than 3.00, and optionally at least 0.40.

Optionally, a ratio of a weight amount of cross-linked polysiloxane structural units and a weight amount of non-cross-linked polysiloxane structural units in the coating is less than 3.00, less than 2.50, less than 1.80, or less than 1.20. The ratio of a weight amount of cross-linked polysiloxane structural units and a weight amount of non-cross-linked polysiloxane structural units in the coating may be at least 0.40, at least 0.60, or at least 0.70. In embodiments, this ratio ranges from 0.40 to 3.00, from 0.60 to 2.50, or from 0.70 to 1.80. The non-cross-linked polysiloxane structural units may help achieve the desired elasticity and low temperature seal functionality that is preferred for the method and containers of this disclosure.

The non-cross-linked polysiloxane structural units may help achieve the desired elasticity and low temperature seal functionality that is preferred for the method and containers of this disclosure. The inventors consider that the cross-linked polysiloxane provides for a polymeric network within which the non-cross-linked polysiloxane remains embedded such that a hybrid coating structure results that combines properties of a cured polymeric network and a liquid silicone oil. This is believed to help achieve a tight seal at low temperatures.

In an embodiment, the coating may comprise both cross-linked polydialkylsiloxane structural units, and non-cross-linked polysiloxane structural units. Specifically, the coating may comprise cross-linked polydialkylsiloxane structural units, and non-cross-linked polysiloxane structural units, wherein the non-cross-linked polysiloxane structural units may be one or more silicone oils, i.e., polydialkylsiloxane structural units, such as polydimethylsiloxane silicone oil.

The coating may comprise more than one type of non-cross-linked polysiloxane structural units, such as at least two types, or at least three types. The types may differ in their viscosities. In embodiments, the coating may comprise at least two non-cross-linked polysiloxane structural units that differ in their viscosities. In some embodiments, the coating comprises high viscosity non-cross-linked polysiloxane structural units having a viscosity of more than 10,000 cSt, and/or low viscosity non-cross-linked polysiloxane structural units having a viscosity of 10,000 cSt, or less. Viscosity may be determined according to DIN EN ISO 3219:1993 using a coaxial-cylinder system at 23 °C and a shear rate of 10 s⁻¹. Optionally, the high viscosity non-cross-linked polysiloxane structural units have a viscosity of at least 15,000 cSt, and/or the low viscosity non-cross-linked polysiloxane structural units have a viscosity of 5,000 cSt, or less.

In an embodiment, the coating comprises the high viscosity non-cross-linked polysiloxane structural units but not necessarily the low viscosity non-cross-linked polysiloxane structural units.

A ratio of a weight amount of the low viscosity non-cross-linked polysiloxane structural units and the high viscosity non-cross-linked polysiloxane structural units (mass_{high} : mass_{low}) may be at least 0.10, at least 0.50, at least 1.00, at least 1.50 or at least 2.00. In some embodiments, this ratio may range up to 5.00, up to 4.00 or up to 3.00. For example, the ratio of a weight amount of the low viscosity non-cross-linked polysiloxane structural units and the high viscosity non-cross-linked polysiloxane structural units may range from 0.10 to 5.00, from 0.50 to 4.00, or from 1.00 to 3.00.

The cross-linked polysiloxane structural units, the low viscosity non-cross-linked polysiloxane structural units, and/or the high viscosity non-cross-linked polysiloxane structural units, may comprise or consist of dialkylsiloxane monomeric units, in particular dimethylsiloxane monomeric units.

Optionally, the low viscosity non-cross-linked polysiloxane structural units have a weight average molecular weight of 1,200 to 30,000 g/mol, and/or the high viscosity non-cross-linked polysiloxane structural units have a weight average molecular weight of 15,000 to 300,000 g/mol. In an embodiment, the high viscosity non-cross-linked polysiloxane structural units have a weight average molecular weight of 32,000 to 210,000 g/mol, or from 100,000 to 150,000 g/mol. In an embodiment, the low viscosity non-cross-linked polysiloxane structural units have a weight average molecular weight of 5,000 to 25,000 g/mol, or from 10,000 to 20,000 g/mol.

In embodiments, the low viscosity non-cross-linked polysiloxane structural units have a weight average molecular weight of at least 1,200 g/mol, at least 5,000 g/mol, or at least 10,000 g/mol. The weight average molecular weight may range up to 30,000 g/mol, up to 25,000 g/mol, or up to 20,000 g/mol.

In embodiments, the high viscosity non-cross-linked polysiloxane structural units have a weight average molecular weight of at least 15,000 g/mol, at least 32,000 g/mol, or at least 100,000 g/mol. The weight average molecular weight may range up to 300,000 g/mol, up to 210,000 g/mol, or up to 150,000 g/mol.

The weight average molecular weight may be determined with gel permeation chromatography (GPC) according to DIN EN ISO 13885-1:2021-11 using a polystyrene standard as the reference and toluene as the eluent.

In embodiments, the coating composition may have the following composition in percent by weight:

| | |
|---|---|
| one or more cross-linkable polysiloxane compounds | 3.0 % to 20.0 % |
| one or more non-cross-linkable polysiloxane compounds | 2.0 % to 15.0 % |
| one or more cross-linking polysiloxane compounds | 0.10 % to 1.50 % |
| one or more catalysts | 0.03 % to 0.50 % |
| one or more diluents | 65.0 % to 92.0 % |

In embodiments, the coating composition may have the following composition in percent by weight:

| | |
|---|---|
| one or more cross-linkable polysiloxane compounds | 8.0 to 20.0 % |
| one or more non-cross-linkable polysiloxane compounds | 2.0 to 10.0 % |
| one or more cross-linking polysiloxane compounds | 0.10 to 1.00 % |
| one or more catalysts | 0.03 to 0.50 % |
| one or more diluents | 69.0 to 90.0 % |

In embodiments, the coating composition may have the following composition in percent by weight:

| | |
|---|---|
| a vinyl-functionalized polysiloxane as a cross-linkable polysiloxane compound | 3.0 % to 20.0 % |
| one or more non-cross-linkable polysiloxane compounds | 2.0 % to 15.0 % |
| a copolymer having dimethylsiloxane and methylhydrosiloxane monomer units as a cross-linking polysiloxane compound | 0.10 % to 1.50 % |
| one or more Pt-containing catalysts | 0.03 % to 0.50 % |
| one or more diluents | 65.0 % to 92.0 % |

Without wishing to be bound by this theory, the inventors consider curing temperature of the coating composition relevant for achieving a tight seal. The coating may be cured at a curing temperature below 150 °C, below 125 °C, or below 110 °C. Too high a curing temperature may yield a coating having a low elasticity. On the other hand, too low a curing temperature may not be sufficient for a good sealing performance either. Thus, in embodiments, the curing temperature may be 50 °C or more, 60 °C or more, or 70 °C or more. Notably, the curing temperature is the effective temperature at the coating composition. It must not be confused with a nominal oven temperature. The oven temperature may be much higher than the curing temperature because there may be insufficient time for the whole oven to equilibrate at the nominal temperature during curing time. Optionally, the coating may be cured at 50 °C to below 150 °C, from 60 °C to below 125 °C, or from 70 °C to below 110 °C. A preferred range is from 50 °C to < 110 °C.

In embodiments, the coating is obtainable or obtained by applying a coating composition as disclosed herein to at least parts of a surface of the glass container (e.g., an inner surface and/or a nestling surface), and curing the coating composition on the surface, wherein a curing temperature of the coating composition is less than 150 °C, particularly at from 50 °C to < 110 °C.

In embodiments, the coating on the glass container may have been cured at a temperature of less than 150 °C, or less than 125 °C, and/or of 50 °C or more, or 60 °C or more.

The inventors found that both coating thickness and curing temperature influence performance of a seal at very low temperatures. Particularly, curing temperatures should not be too high in order to avoid undesired chemical reactions, for example excessive crosslinking or polymerization. In some instances, a coating cured at very high temperatures was not sufficiently elastic at low temperatures in order to maintain a tight seal, further the glide force is increased at high curing temperatures.

Optionally, the curing temperature may be held for at least 10 seconds, at least 30 seconds, at least 45 seconds or at least 60 seconds. In embodiments, the curing temperature is held for up to 3000 seconds, up to 300 seconds, or up to 180 seconds. Too long a curing time at high temperatures may impair elasticity and glide force.

Optionally, the coating may be used without a separate curing step. As the glass containers generally are sterilized at elevated temperatures before assembly with the stopper, this temperature treatment can be used for simultaneously curing the coating on the glass surface. When using this option, the curing temperature of the coating will typically be closer to the lower end of the above-mentioned range. The inventors have found that this combined sterilization and curing step does not lead to inferior results regarding CCI when compared to a separate curing step using the same curing temperature.

For a suitable sealing efficacy, it will often be sufficient to apply the coating on an area where a tight seal is desired, i.e., a contact area of inner surface and stopper, and/or of a nestling surface and cap. The coating may be disposed on at least 25 %, or at least 50 % of the inner surface of the hollow cylindrical body (area by area). However, the coating may also have a beneficial effect on the gliding properties of a stopper on the inner surface of the hollow cylindrical body. Therefore, in some embodiments, the coating is disposed on at least 65 %, or at least 85 % of the inner surface of said hollow cylindrical body (area by area). Optionally, the coating is disposed on at least 90 %, or essentially all of the inner surface of said hollow cylindrical body. Alternatively or additionally, the coating is disposed on at least 65 %, or at least 85 % of the nestling surface of the glass container (area by area). Optionally, the coating is disposed on at least 90 %, or essentially all of the nestling surface.

### Stopper

The stopper has a body which may have at least one annular protrusion and a circumferential surface. The stopper may also have at least two annular protrusions. In an embodiment, the stopper may have from one to five annular protrusions, such as from two to four annular protrusion. In specific embodiments, the stopper may have one, two, three, four, or five annular protrusions.

The "circumferential surface" is the surface of the stopper that faces towards the inner surface of the hollow cylindrical body when the stopper is disposed in the hollow cylindrical body. The circumferential surface includes the surface of any annular protrusions. If the stopper is coated, the surface of the coating that faces the inner surface of the hollow cylindrical body is part of or constitutes the circumferential surface. The "contact surface" is the part of the circumferential surface that touches the inner surface of the hollow cylindrical body when the stopper is inserted in the hollow cylindrical body (e.g., at 20 °C). In this disclosure, it may be beneficial if the stopper is not coated. It was found that non-coated stoppers may form a tighter seal at low temperatures than a coated stopper. In an embodiment, the stopper does not comprise a fluorinated polymer coating.

An "annular protrusion" is a portion of the stopper that has a greater than average diameter, measured perpendicular to the longitudinal axis of the hollow cylindrical body, such as the barrel. The annular protrusions touch the inner surface of the hollow cylindrical body so as to seal the junction between stopper and hollow cylindrical body. Any portion of the stopper having a greater than average diameter, but not touching the inner surface of the hollow cylindrical body to an extent of at least 80.0 %, 90.0 %, 99.9 % or 100 % during movement of the stopper in distal direction is not considered an "annular protrusion".

At least one and preferably all of the annular protrusions may have a diameter that exceeds the inner diameter of the hollow cylindrical body. Preferably, the diameter of at least one and preferably all of the annular protrusions exceed the inner diameter of the hollow cylindrical body by at least 0.05 mm, or at least 0.1 mm, or at least 0.15 mm. The outer diameter of the annular protrusion may be equivalent to the outer diameter of the stopper. The diameter is measured perpendicular to the hollow cylindrical body's longitudinal axis.

Annular protrusions help keeping the stopper in the intended position within the hollow cylindrical body, stabilize its orientation in the proximal-distal direction, and thereby influence the BLF and GF values of the container. Further, the annular protrusions seal the junction between stopper and inner surface of the hollow cylindrical body.

The stopper may optionally feature one or more trailing ribs. A "trailing rib" is a portion of the stopper that has a greater than average diameter, measured perpendicular to the longitudinal axis of the hollow cylindrical body. However, the trailing rib has a smaller diameter than an annular protrusion so that it does not touch the hollow cylindrical body's inner surface to a significant extent, when the stopper is moved in the proximal-distal direction. Such trailing ribs may serve the purpose of stabilizing the stoppers orientation within the hollow cylindrical body, without effectively sealing the junction between stopper and inner surface. Trailing ribs do usually not significantly influence BLF and GF because their contact with the inner surface is limited, if any.

The stopper may be coated with a coating. The coating may be a polymer. In an embodiment, the coating comprises a resin, such as a fluorinated polymer such as a polymer selected from the group consisting of polytetrafluoroethylene (PTFE), densified expanded polytetrafluoroethylene (ePTFE), tetrafluoroethylene (TFE), tetrafluoroethylene-perfluoroethylene copolymer, tetrafluoroethylene-hexafluoropropylene copolymer, tetrafluoroethylene-ethylene copolymer, trichlo-rotrifluoroethylene, poly-vinylidene fluoride, polyvinyl fluoride, perfluoropropylvinylether, per-fluoroalkoxy polymers, as well as copolymers, blends and combinations thereof. The coating may also be formed by layers comprising polyethylene, polypropylene, polyparaxylxylene, polylactic acid, as well as copolymers, blends, and combinations thereof. A PTFE coating is a preferred coating option. These coatings reduce the coefficient of friction of the stopper's circumferential surface on the inner surface of the hollow cylindrical body. In embodiments, at least the parts of the stopper's circumferential surface that are supposed to be in contact with the hollow cylindrical body's inner surface will be coated.

The stopper may have an elastomeric body with an at least 10 MPa yield stress measured according to ISO 527-2:2012(E) and/or a low coefficient of sliding friction below 0.23 against steel measured according to DIN EN ISO 8295/2004-10. The stopper may be made of thermoplastic elastomers and/or rubbers, such as natural or synthetic rubbers. Suitable rubber materials may be selected from the group consisting of butyl rubbers, halogenated butyl rubbers, acrylonitrile-butadiene rubbers, isoprene rubbers, neoprene rubbers, butadiene rubbers, styrene-butadiene rubbers, ethylene-propylene rubbers, isoprene-isobutylene rubbers, nitrile rubbers, and combinations and mixtures thereof. In one embodiment, the stopper is made of bromobutyl rubber. Particularly, the body of the stopper may be made of the above-listed rubbers and/or thermoplastic elastomers.

The body may be coated with a resin as described above. The stopper coating may have a thickness of less than 1 mm, particularly from 0.5 µm to 200 µm, particularly from 10 µm to 125 µm, or from 30 µm to 100 µm. These thicknesses have been proven to be easily applied and sufficient for the desired effect on the friction.

The circumferential surface of the stopper may have a water contact angle of at least 100°, or even at least 110°. The circumferential surface of the stopper may be superhydrophobic. Using superhydrophobic stoppers in the containers of this invention contributes to the beneficial BLGF values due to the low coefficient of sliding friction in combination of a low adhesion disposition.

The circumferential surface of the stopper and the inner surface of the hollow cylindrical body may at least partially contact each other in a contact area. The contact area is sometimes also referred to as the sealing area. In embodiments, the contact area will be at least 8 mm² and at most 48 mm². The contact area may be 8-48 mm² or 10-40 mm², 15-30 mm², 16-24 mm². In the case of plural annular protrusions each protrusion contributes to the contact area. A minimum contact area will be useful to achieve sufficient sealing. If the contact area is too high, BLGF values may increase too much.

The system may comprise a stopper having a Shore A hardness of not more than 70. Shore A hardness may be tested using the method of ISO 7619-1 (2012-02, 1sec indentation). Shore A hardness may be at least 35, at least 40, or at least 45. Optionally, it may be up to 65, or up to 60. In embodiments, Shore A hardness reaches from 35 to 70, from 40 to 65, or from 45 to 60.

The stopper may have a density of at least 1.200 g/cm³, at least 1.250 g/cm³, or at least 1.300 g/cm³. Optionally, the density is at most 1.450 g/cm³, at most 1.400 g/cm³, or at most 1.385 g/cm³. In embodiments, the density ranges from 1.200 g/cm³ to 1.450 g/cm³, from 1.250 g/cm³ to 1.400 g/cm³, or from 1.300 g/cm³ to 1.385 g/cm³.

In embodiments, when inserted in the hollow cylindrical body of the glass container, a stopper compression ratio of the at least one stopper defined as (Dₛ - D_{b}) / Dₛ, with Dₛ = outer diameter of the uncompressed stopper and D_{b} = inner diameter of the hollow cylindrical body, may be
- 8.0 % or more, or 10.0 % or more, or 12.0 % or more, or 14.0 % or more, or 15.0 % or more; and/or
- 18.0 % or less, or 17.5 % or less, 17.0 % or less, 16.5 % or less, 16.0 % or less.

The stopper compression ratio may be in a range of from 8.0 % to 18.0 %, or from 10.0 % to 17.5 %, or from 12.0 % to 17.0 %, or from 14.0 % to 16.5 %, or from 15.0 % to 16.0 %. The stopper compression ratio may for example be determined by measuring the outer diameter of the uncompressed stopper and the inner diameter of the hollow cylindrical body by means of a caliper. Optionally, the stopper compression ratio is limited to not more than 35.0 %, not more than 30.0 %, or not more than 25.0 %.

The inventors have found that in the case of glass containers another important factor for achieving CCI at such low temperatures are the relaxation kinetics, particularly at temperatures below the glass transition temperature of the stopper material. When cooling the filled container for storing, there will occur different forces. There will be radial forces acting on the cylindrical body / coating / stopper interface due to i) shrinkage of stopper with decreasing temperature, ii) shrinkage of coating with decreasing temperature, and iii) inhibited or slowed down relaxation of the stopper below its glass transition temperature. Further, there will be shear forces acting on the coating interface due to stopper movement caused by the freezing of the filling medium of the container, particularly with aqueous solutions.

At temperatures above the glass transition temperature of the stopper material, the reduction of the stopper diameter may be compensated by the relaxation of the stopper compression initially applied by the cylindrical body of the container. However, at temperatures below the glass transition temperature, the mobility of the elastomer molecules is very strongly restricted, and the material behaves purely energy-elastic. With decreasing temperature, dimensional recovery becomes very slow. It has been surprisingly found that a stopper with an initially higher compression recovers faster its size even at low temperatures. Hence, the stopper compression ratio is an additional factor for further improving the CCI at -80 °C and below. The amount of the stopper compression ratio has to be balanced between improving relaxation of the shrinking stopper and usability at room temperature which includes keeping the total glide force variation (TGFV) very low and reducing break loose force. Too high a ratio will strongly increase break loose and glide forces. Excessive compression might even damage the coating when the stopper is moved in the cylindrical body of the container.

In embodiments, it might be preferred that the total length of the hollow cylindrical body, especially measured along the axial extension of the barrel, is
(i) 40 mm or more, preferably 45 mm or more, preferably 50 mm or more, preferably 55 mm or more, preferably 60 mm or more, preferably 65 mm or more, preferably 70 mm or more, preferably 75 mm or more, preferably 80 mm or more,
(ii) 80 mm or less, preferably 75 mm or less, preferably 70 mm or less, preferably 65 mm or less, preferably 60 mm or less, preferably 55 mm or less, preferably 50 mm or less, preferably 45 mm or less, preferably 40 mm or less,
   and/or
(iii) between 40 mm and 80 mm, preferably between 40 mm and 60 mm, especially between 45 mm and 55 mm, particularly between 45 mm and 50 mm, or between 60 mm and 70 mm, particularly between 63 mm and 67 mm.

For example, the total length of the hollow cylindrical body can be 65.7 mm or 48.4 mm.

In embodiments, the inside diameter of the hollow cylindrical body may be
(i) 5.0 mm or more, or 6.0 mm or more, or 7.0 mm or more, or 8.0 mm or more, or 9.0 mm or more,
(ii) 10.0 mm or less, or 9.0 mm or less, or 8.0 mm or less, or 7.0 mm or less, or 6.0 mm or less, or 5.0 mm or less, and/or
(iii) between 5.0 mm and 10.0 mm, or between 5.0 mm and 7.0 mm, especially between 5.0 mm and 6.0 mm or between 6.0 mm and 7.0 mm, particularly between 5.5 mm and 6.0 mm, such as 5.85 mm, or between 6.0 mm and 6.5 mm, such as 6.35 mm, or between 7.0 mm and 9.0 mm, especially between 8.0 mm and 9.0 mm, particularly between 8.5 mm and 9.0 mm, such as 8.65 mm.

For example, the inside diameter of the hollow cylindrical body can be 5.85 mm or 6.35 mm or 8.65 mm.

In embodiments, the outside diameter of the stopper may be
(i) 6.0 mm or more, or 7.0 mm or more, or 8.0 mm or more, or 9.0 mm or more, or 10.0 mm or more,
(ii) 11.0 mm or less, or 10.0 mm or less, or 9.0 mm or less, or 8.0 mm or less, or 7.0 mm or less, or 6.0 mm or less,
   and/or
(iii) between 6.0 mm and 11.0 mm, or between 6.0 mm and 8.0 mm, especially between 6.5 mm and 7.5 mm, particularly between 6.75 mm and 7.25 mm, such as 6.9 mm or 7.0 mm.

For example, the outside diameter of the stopper can be 6.9 mm or 7.0 mm.

### Break loose and glide forces

The glass container according to aspects of this disclosure may have a standardized glide force of not more than 5.0 N.

The glide force indicates the force needed to push a stopper in the hollow cylindrical body, whereas the break loose force indicates the forces needed to cause an initial movement of the stopper within the hollow cylindrical body. A "standardized glide force" is a glide force (GF) measured at standard conditions. Likewise, a "standardized break loose force" is a break loose force (BLF) measured at standard conditions. Standard conditions include a standard stopper, i.e., a Datwyler FM257/2 stopper made of bromobutyl rubber having a hardness of 52 Shore A, and density of 1.355 g/cm³, available from Datwyler Pharma Packaging International NV, Indus-trieterrein Kolmen 1519, BE-3570 Alken, Belgium). BLF and GF can be measured in one take. A test for BLF and GF may be referred to as "BLGF" test. A "specific" BLF or GF relates to the break loose or glide force measured in a certain system, i.e., including the system's stopper, instead of the standard stopper. Other than that, the measurement is the same as for the standard test.

The standardized BLGF test is conducted on a universal testing machine at room temperature, i.e., 20 °C. A standardized BLGF testing device with a 50 N test cup is used for this purpose. The samples were fixed in vertical orientation in a universal testing machine model 106, 2 kN from TesT AG, CH-6331 Hünenberg, Switzerland.

The BLF is the force needed to move the stopper from its original position. The GF is the force needed to keep the stopper moving after breaking it loose.

The containers are filled with water for injection. After filling the specimens, they are either stored or tested immediately, depending on the test purpose. The specimens are tested without needles.

The specimens are inserted into the holder and the pressure stamp is moved towards the stopper at a rate of 20 mm/min. Once a force of 0.25 N is measured the machine switches to the test rate of 100 mm/min and starts recording the data. The experiment ends when the measured force exceeds 35 N, which is usually the case when the distal end of the hollow cylindrical body is reached.

The BLF is the highest force measured within the first 4 mm of stopper movement. The GF values are measured within a test range starting after 4 mm of movement and ending 10 mm before reaching the distal end of the barrel, the GF according to this disclosure is the highest glide force measured in this experiment.

The glass containers of this invention may exhibit a standardized BLF of not more than 12.0 N. In some embodiments, the standardized BLF may be limited to upper limits of 9.0 N, 8.0 N, 7.0 N, 6.0 N, 5.0 N or even 4.0 N. The standardized BLF may be at least 0.1 N, at least 0.5 N, or at least 1.0 N so as to avoid any unintended movement of the stopper.

The glass container exhibits a ratio of the standardized BLF relative to the standardized GF of BLF/GF > 1.30. Optionally, the ratio of the standardized BLF relative to the standardized GF is characterized by BLF/GF ≤ 3.0, in particular even after storing the glass container at -80 °C for 168 hours. In embodiments, the ratio BLF/GF is > 1.40, > 1.50, or even > 1.60 for the containers of this disclosure. In some embodiments, the ratio BLF/GF may be < 2.5, < 2.2, < 2.0, or even < 1.9 for the containers of this invention after storing the glass container at -80 °C for 168 hours ("low temperature stored container"). Particularly, the relative difference in the ratios BLF/GF of low temperature stored containers, and non-stored containers (BLF/GF_{-80 °C} - BLF/GF₀)/ BLF/GF_{-80 °C} may be less than 10 %, preferably less than 5 %. This means that the impact of a freeze-thaw cycle on the ratio BLF/GF is low. On the other hand, a BLF/GF ratio as discussed above is rather high, which means that it is comparably difficult to break loose the stopper from its initial position. This may be due to an interaction between coating and stopper. A higher BLF is useful for the stopper to stay in its initial position at low temperatures, i.e., when the pharmaceutical composition expands due to freezing.

The standardized GF of the glass containers of this disclosure may be < 7.5 N, < 6.5 N, < 5.5 N, < 4.5 N, < 3.5 N, or even < 2.5 N. Optionally, the relative difference in the standardized BLF of low temperature stored containers and non-stored containers (BLF_{-80 °C} - BLF₀)/ BLF_{-80 °C} is less than 25 %, < 20 %, < 15 %, < 10 %, or even < 5 %. The relative difference in the GF of low temperature stored containers, and non-stored containers (GF_{-80 °C} - GF₀)/ GF_{-80 °C} is less than 25 %, < 20 %, < 15 %, < 10 %, or even < 5 %. Keeping GLF comparably low helps fully expel the contents of the container with the stopper after low temperature storage.

A suitable glide force and break loose force is relevant for a convenient use of glass containers of this disclosure. Generally, a tight seal corresponds to a high break loose and/or glide force. Some glass containers of this disclosure exhibit standardized break loose and glide forces that are remarkably low. However, a sufficiently high break loose force may be beneficial for inhibiting undesired stopper movement during storage.

Optionally, the glass container according to this disclosure has a standardized glide force of at least 0.5 N.

In order to safeguard a tight seal by inhibiting stopper movement during low temperature storage, glass containers of this disclosure may have a standardized break loose force that exceeds the container's standardized glide force by at least 30 %, at least 60 %, at least 100 %, or at least 200 %.

In one embodiment, a container of this disclosure has a specific break loose force that is at least 600 % larger than a specific glide force and the specific break loose force is at least 4.0 N, or at least 4.9 N.

### Glass composition

The glass of the glass container is not particularly limited. Preferably, the glass is a borosilicate glass, an aluminosilicate glass, a lithium-aluminosilicate (LAS) glass, more preferably a borosilicate glass.

In an embodiment, the composition of the glass comprises, in mass-%:
SiO₂: 30 % to 98 %, preferably 50 % to 90 %, more preferably 70.0 % to 74.0 %; and/or
B₂O₃: 0 % to 30 %, preferably 3 % to 20 %, more preferably 7.0 % to 16.0 %; and/or
Al₂O₃: 0 % to 30 %, preferably 1 % to 15 %, more preferably 3.0 % to 6.5 %; and/or
X₂O: 0 % to 30 %, preferably 1 % to 15 %, more preferably 2.0 % to 7.2 %, wherein X is selected from Na, K, Li, preferably X is Na and/or K; and/or
YO: 0 % to 30 %, preferably 0.1 % to 5 %, more preferably 0.5 % to 1.0 %, wherein Y is selected from Ca, Mg, Ba, preferably Y is Ca and/or Mg.

More preferably, the composition of the glass consists of, in mass-%:
SiO₂: 30 % to 98 %, preferably 50 % to 90 %, more preferably 70.0 % to 74.0 %;
B₂O₃: 0 % to 30 %, preferably 3 % to 20 %, more preferably 7.0 % to 16.0 %;
Al₂O₃: 0 % to 30 %, preferably 1 % to 15 %, more preferably 3.0 % to 6.5 %;
X₂O: 0 % to 30 %, preferably 1 % to 15 %, more preferably 2.0 % to 7.2 %, wherein X is selected from Na, K, Li, preferably X is Na and/or K;
YO: 0 % to 30 %, preferably 0.1 % to 5 %, more preferably 0.5 % to 1.0 %, wherein Y is selected from Ca, Mg, Ba, preferably Y is Ca and/or Mg.

In another preferred embodiment, the composition of the glass comprises, in mass-%:
SiO₂: 20 % to 98 %, preferably 40 % to 75 %, more preferably 50 % to 65 %; and/or
B₂O₃: 0 % to 30 %, preferably 1 % to 15 %, more preferably 3 % to 9 %; and/or
Al₂O₃: 0 % to 30 %, preferably 10 % to 20 %, more preferably 13 % to 18 %; and/or
X₂O: 0 % to 30 %, preferably 0 % to 5 %, more preferably 0 % to 3 %, wherein X is selected from Na, K, Li, preferably X is Na and/or K; and/or
YO: 0 % to 50 %, preferably 0.1 % to 40 %, more preferably 10 % to 35 %, wherein Y is selected from Ca, Mg, Ba, preferably Y is Ca and/or Mg.

More preferably, the composition of the glass consists of, in mass-%:
SiO₂: 20 % to 98 %, preferably 40 % to 75 %, more preferably 50 % to 65 %;
B₂O₃: 0 % to 30 %, preferably 1 % to 15 %, more preferably 3 % to 9 %;
Al₂O₃: 0 % to 30 %, preferably 10 % to 20 %, more preferably 13 % to 18 %;
X₂O: 0 % to 30 %, preferably 0 % to 5 %, more preferably 0 % to 3 %, wherein X is selected from Na, K, Li, preferably X is Na and/or K;
YO: 0 % to 50 %, preferably 0.1 % to 40 %, more preferably 10 % to 35 %, wherein Y is selected from Ca, Mg, Ba, preferably Y is Ca and/or Mg.

The volume of the glass container is not particularly limited. Preferably, the brimful volume of the container is 0.1 ml to 1000 ml, preferably, 0.5 ml to 500 ml, more preferably 1 ml to 250 ml, more preferably 2.0 ml to 30.0 ml, more preferably 2.0 ml to 15.0 ml, more preferably about 1.0 ml, 2.0 ml, 3.0 ml, 4.0 ml, 5.0 ml, 6.0 ml, 7.0 ml, 8.0 ml, 9.0 ml, 10.0 ml, 11.0 ml, 12.0 ml, 13.0 ml, 14.0 ml or 15.0 ml; more preferably 5.0 ml to 15.0 ml.

In one embodiment, the glass of the glass container has a glass composition comprising 50 wt.-% to 90 wt.-% SiO₂, and 3 wt.-% to 25 wt.-% B₂O₃.

In one embodiment, the glass of the glass container has a glass composition comprising aluminosilicate, optionally comprising 55.0 wt.-% to 75.0 wt.-% SiO₂, and 11.0 wt.-% to 25.0 wt.-% Al₂O₃.

In one embodiment, the glass of the glass container has a glass composition comprising 70.0 wt.-% to 81.0 wt.-% SiO₂, 1.0 wt.-% to 10.0 wt.-% Al₂O₃, 6.0 wt.-% to 14.0 wt.-% B₂O₃, 3.0 wt.-% to 10.0 wt.-% Na₂O, 0.0 wt.-% to 3.0 wt.-% K₂O, 0.0 wt.-% to 1.0 wt.-% Li₂O, 0.0 wt.-% to 3.0 wt.-% MgO, 0.0 wt.-% to 3.0 wt.-% CaO, and 0.0 wt.-% to 5.0 wt.-% BaO.

In one embodiment, the glass of the glass container has a glass composition comprising 72.0 wt.-% to 82.0 wt.-% SiO₂, 5.0 wt.-% to 8.0 wt.-% Al₂O₃, 3.0 wt.-% to 6.0 wt.-% B₂O₃, 2.0 wt.-% to 6.0 wt.-% Na₂O, 3.0 wt.-% to 9.0 wt.-% K₂O, 0.0 wt.-% to 1.0 wt.-% Li₂O, 0.0 wt.-% to 1.0 wt.-% MgO, and 0.0 wt.-% to 1.0 wt.-% CaO.

In one embodiment, the glass of the glass container has a glass composition comprising 60.0 wt.-% to 78.0 wt.-% SiO₂, 7.0 wt.-% to 15.0 wt.-% B₂O₃, 0.0 wt.-% to 4.0 wt.-% Na₂O, 3.0 wt.-% to 12.0 wt.-% K₂O, 0.0 wt.-% to 2.0 wt.-% Li₂O, 0.0 wt.-% to 2.0 wt.-% MgO, 0.0 wt.-% to 2.0 wt.-% CaO, 0.0 wt.-% to 3.0 wt.-% BaO, and 4.0 wt.-% to 9.0 wt.-% ZrO₂.

In one embodiment, the glass of the glass container has a glass composition comprising 50.0 wt.-% to 70.0 wt.-% SiO₂, 10.0 wt.-% to 26.0 wt.-% Al₂O₃, 1.0 wt.-% to 14.0 wt.-%. B₂O₃, 0.0 wt.-% to 15.0 wt.-% MgO, 2.0 wt.-% to 12.0 wt.-% CaO, 0.0 wt.-% to 10.0 wt.-% BaO, 0.0 wt.-% to 2.0 wt.-% SrO, 0.0 wt.-% to 8.0 wt.-% ZnO, and 0.0 wt.-% to 2.0 wt.-% ZrO₂.

In one embodiment, the glass of the glass container has a glass composition comprising 55.0 wt.-% to 70.0 wt.-% SiO₂, 11.0 wt.-% to 25.0 wt.-% Al₂O₃, 0.0 wt.-% to 10.0 wt.-% MgO, 1.0 wt.-% to 20.0 wt.-% CaO, 0.0 wt.-% to 10.0 wt.-% BaO, 0.0 wt.-% to 8.5 wt.-% SrO, 0.0 wt.-% to 5.0 wt.-% ZnO, 0.0 wt.-% to 5.0 wt.-% ZrO₂, and 0.0 wt.-% to 5.0 wt.-% TiO₂.

In one embodiment, the glass of the glass container has a glass composition comprising 65.0 wt.-% to 72.0 wt.-% SiO₂, 11.0 wt.-% to 17.0 wt.-% Al₂O₃, 0.1 wt.-% to 8.0 wt.-% Na₂O, 0.0 wt.-% to 8.0 wt.-% K₂O, 3.0 wt.-% to 8.0 wt.-% MgO, 4.0 wt.-% to 12.0 wt.-% CaO, and 0.0 wt.-% to 10.0 wt.-% ZnO.

In one embodiment, the glass of the glass container has a glass composition comprising 64.0 wt.-% to 78.0 wt.-% SiO₂, 4.0 wt.-% to 14.0 wt.-% Al₂O₃, 0.0 wt.-% to 4.0 wt.-% B₂O₃, 6.0 wt.-% to 14.0 wt.-% Na₂O, 0.0 wt.-% to 3.0 wt.-% K₂O, 0.0 wt.-% to 10.0 wt.-% MgO, 0.0 wt.-% to 15.0 wt.-% CaO, 0.0 wt.-% to 2.0 wt.-% ZrO₂, and 0.0 wt.-% to 2.0 wt.-% TiOz.

In an embodiment, the glass of the glass container has an average linear coefficient of thermal expansion measured in the range of 20°C to 300°C (CTE) between 3.0 * 10⁻⁶ K⁻¹ and 8.0 * 10⁻⁶ K⁻¹, or between 3.5 * 10⁻⁶ K⁻¹ and 7.0 * 10⁻⁶ K⁻¹, or between 4.0 * 10⁻⁶ K⁻¹ and 6.0 * 10⁻⁶ K⁻¹. Optionally the CTE may be less than 5.2 * 10⁻⁶ K⁻¹ or less than 5.1 * 10⁻⁶ K⁻¹. In some embodiments, the CTE is limited to no more than 6.9 * 10⁻⁶ K⁻¹ or no more than 5.9 * 10⁻⁶ K⁻¹. The CTE may be measured according to DIN ISO 7991:1987.

### Headspace

In embodiments, in addition to the pharmaceutical composition a gas may be filled into the glass container, and a volume enclosed by the glass container which is occupied by the gas may be defined as headspace of the glass container.

The headspace may be variable depending on the ambient temperature, the ambient pressure, or the like. This is because the headspace corresponds to the volume of the gas within the hollow cylindrical body of the glass container which volume may be subject to changes. For example, the volume of the gas depends on the environmental conditions and/or on the volume of the pharmaceutical composition within the hollow cylindrical body.

In one embodiment it might be preferred that, at room temperature, the volume occupied by the headspace is 1 % or more of the volume occupied by the pharmaceutical composition.

The inventors have found that CCI at -80 °C or below may be further enhanced if a gas volume of appropriate size is provided within the glass container. It turned out that this allows a change of the volume of the pharmaceutical composition without affecting the integrity of the container during cooling or thawing. This is because the proposed headspace makes the system resistant to the expansion or retraction of the pharmaceutical composition when the composition is cooled, especially below the freezing point of the pharmaceutical composition. The same applies when the system is thawed and the pharmaceutical composition expands or retracts as well. This way, the above-mentioned radial forces acting on the coating interface can be minimized or eliminated.

In other words, the method including a headspace achieves a further significant reduction of the risk that due to a change of volume of the pharmaceutical composition during cooling or thawing, the stopper moves or the container leaks. The force applied to the stopper is reduced in that the headspace is variable, i.e., that the volume of the gas can be changed, e.g., compressed or expanded, by the space the composition occupies. Hence, an uncontrolled movement of the stopper which may cause damage to the coating interface can be prevented.

In embodiments, the gas may be or may comprise one or more of air, CO₂, N₂, Ar, and/or O₂.

If the gas is air, CO₂, N₂, Ar, and/or O₂, a particular cheap provision of the method is possible.

In embodiments, the glass container may be oriented vertically after filling and before cooling, such that the headspace of the glass container is located adjacent to the stopper.

The inventors have found that the position of the headspace may have a positive impact on the CCI. They theorize that freezing of the pharmaceutical composition begins at the gas/liquid interface. Hence, if the headspace is positioned distal to the stopper, the freezing and consequently expanding liquid exerts an increasing pressure on the stopper during the freezing procedure. If the created force becomes higher than the break loose force of the stopper, it will start moving, or if the forces on the coating interface become too high, it may be damaged and CCI may be lost. When the headspace is positioned adjacent to the stopper, the increasing force is mainly directed towards the less critical cap side of the container, such as the cone with a tip cap of a syringe, which compensates this pressure with the cap simultaneously shrinking further onto the cone. The stopper is protected by the shrinking gas volume from an excessive pressure.

In embodiments, the headspace may have a cylindrical volume domain portion which volume domain portion has a specific diameter equal to the inside diameter of the barrel and a specific height, which specific height preferably is measured from the central point of the stopper to the surface of the pharmaceutical composition facing the stopper, wherein the specific height has a value of 0.1 mm or more.

Preferably, when measuring the specific height, the container has a vertical orientation with the open end closed by the stopper being at the top.

It is noted that for a stopper with the surface facing the pharmaceutical composition being a flat surface, the volume of the cylindrical volume domain portion might be equal to the volume of the headspace.

It is the surprising finding that a specific height chosen accordingly leads to the beneficial situation that the interaction between an expansion of the pharmaceutical composition and a compression of the gas is such that only reduced or even no movement of the stopper is observed during the freezing and thawing process. The inventors have found that the specific height of at least 0.1 mm constitutes an optimal basis for said interaction.

In embodiments, the headspace may have a cylindrical volume domain portion which volume domain portion has a specific diameter equal to the inside diameter of the hollow cylindrical body and a specific height, which specific height preferably is measured from the central point of the at least one stopper to the surface of the pharmaceutical composition facing the at least one stopper, wherein the specific height has a value of
- 2.0 mm or more, or 3.0 mm or more, or 4.0 mm or more, or 5.0 mm or more, or 6.0 mm or more, or 7.0 mm or more, or 8.0 mm or more, or 9.0 mm or more, or 10.0 mm or more, or 11.0 mm or more, or 12.0 mm or more, or 13.0 mm or more, or 14.0 mm or more, or 15.0 mm or more; and/or
- 15.0 mm or less, or 14.0 mm or less, or 13.0 mm or less, or 12.0 mm or less, or 11.0 mm or less, or 10.0 mm or less, or 9.0 mm or less, or 8.0 mm or less, or 7.0 mm or less, or 6.0 mm or less, or 5.0 mm or less, or 4.0 mm or less, or 3.0 mm or less; and/or
- between 2.0 mm and 15.0 mm, or between 2.0 mm and 10.0 mm, or between 3.0 mm and 9.0 mm, or between 3.0 mm and 5.0 mm, such as 4.0 mm, or between 5.0 mm and 7.0 mm, such as 6.0 mm, or between 7.0 mm and 9.0 mm, such as 8.0 mm.

The specific height can further be specified dependent on the situation the system is used for. The proposed specific height provided preferred results concerning a reduced or even eliminated stopper movement during the freezing and thawing process.

### Ethanol-modified dye-ingress test

In embodiments, the glass container may have a standard ethanol-modified dye-ingress tested container closure integrity (CCI) of at least 168 hours at -80 °C and/or an O₂ headspace analysis tested container closure integrity of at least 1 hour at -96 °C.

In an embodiment, a system comprises the glass container of this disclosure and a stopper wherein the system has a specific ethanol-modified dye-ingress tested container closure integrity (CCI) of at least 168 hours at -80 °C and/or an O₂ headspace analysis tested container closure integrity of at least 1 hour at -96 °C.

Optionally, the system may have a specific ethanol-modified dye-ingress tested container closure integrity of at least 300 hours, at least 600 hours, or at least 1,200 hours at -80 °C. Optionally, the system may have an O₂ headspace analysis tested container closure integrity of at least 2 hours at -96 °C or at least 3 hours at -96 °C.

The specific ethanol-modified dye-ingress tested container closure integrity may be stopper-related and/or tip-related. "Stopper-related" means that the seal formed by the stopper sitting in the hollow cylindrical body is examined for its closure integrity. "Tip-related" means that the seal formed by a cap sitting on a tip-sided opening is examined for its closure integrity. Unlike a standard container closure integrity test, which relies on a standard stopper, plunger and/or cap, the specific ethanol-modified dye-ingress tested container closure integrity is measured using the specific stopper and/or cap that is part of the system under test.

The ethanol-modified dye-ingress container closure integrity test will now be explained with reference to **Figure 2****.**

Figure 2 shows schematically and exemplarily a setup 200 for determining the ethanol-modified dye-ingress closure integrity of a container, at different stages. The setup 200 comprises an immersion arrangement 270 and a container 201 for use with the immersion arrangement 270.

The container 201 is a glass container, e.g., a glass container according to this disclosure, comprising a hollow cylindrical body 210 having two openings, open end 212-1 and open end 212-2 arranged on different sides of the glass container. Each of the openings 212-1, 212-2 is sealed by means of a closure device 230-1, namely a stopper, or a closure device 230-2, namely a cap, respectively. A container volume 205 of container 201 is defined by a portion of the inner wall of the container body 210 and an inner surface of each of the closure devices 230-1, 230-2.

In case of the standard ethanol-modified dye-ingress container closure integrity test, the stopper is a Datwyler FM257, and the cap is a West W7025 in a Luer Lock SRC rigid cap (as disclosed for example in EP 3 569 272 A1). In case of the specific ethanol-modified dye-ingress container closure integrity test, the stopper is the stopper of the system in question, and the cap, if any, is the cap of the system in question.

In the shown example, container 201 is a medical glass syringe or a pharmaceutical glass cartridge. The test can be performed on other types of glass containers in the same way. Open end 212-1 at a proximal end of the container is sealed by a closure device in the form of a stopper 230-1. Open end 212-2 at a distal end of the container is arranged in a tip of the container 201, and it is sealed by a closure device in the form of a cap 230-2.

For the purpose of this test, container volume 205 is filled with air at 1 atm pressure and sealed using the appropriate closure device. The immersion arrangement 270 comprises an immersion device 272. In the immersion device 272, a reservoir is provided which contains an ambient liquid 274. The immersion device 272 further includes conditioning facilities for adjusting and holding a temperature of the ambient liquid 274 in accordance with the testing protocol.

At the beginning of the test, the container volume 205 is entirely filled with air. In addition, the container 201, including the gas inside the container volume 205, is conditioned at 20 °C. The ambient liquid 274 is conditioned at -80 °C. The ambient liquid 274 is ethanol with a fluorescein dye (fluorescein disodium at 1 g/l).

As shown in Fig. 2, b), in a next step, the container 201 is entirely submerged in the ambient liquid 274. Whilst submerged in the ambient liquid 274, the container 201 will gradually adopt a temperature at least close to -80 °C. The absolute heat capacity of the container 201 is small relative to the absolute heat capacity of the ambient liquid 274. In this way, temperature variations of the ambient liquid 274 due to the immersion of the container 201 are minimal. When the temperature of the container 201 sinks due to the immersion in the ambient liquid 274, a temperature of the gas in the container volume 205 will sink correspondingly. Hence, the gas in the container volume 205 will contract, and a gas pressure within the container volume 205 will drop below the ambient pressure.

The container 201 remains in the ambient liquid 274 during at least a predetermined period of time, starting from the moment the container is fully immersed in the ambient liquid 274, and ending when the container 201 is taken out of the ambient liquid 274 and allowed to equilibrate at ambient temperature (preferably 20 °C).

Fig. 2, c), shows schematically two possible outcomes once the container 201 has been removed from the ambient liquid 274. In the upper example, i), an amount L of the ambient liquid is detected inside the container volume 205. In particular, an amount L of ambient liquid 274 has transgressed a boundary of the container 201 in the region of the tip. This indicates the presence of a leak in the region of the tip of the container 201 under the conditions applied in previous stages a) and b). In the lower example, ii), no parts of the ambient liquid 274 are detected inside the container 201. This indicates tightness of the container 201 under the conditions applied in previous stages a) and b).

A container is considered to have passed the test, if no ambient liquid is detected inside the container, which includes that no ambient liquid is found between annular protrusions of the stopper, or otherwise between closure device and coated surface.

### Container closure integrity test using headspace analysis

Headspace analysis is a deterministic container closure integrity (CCI) test. Non-destructive headspace analysis as a CCI test method is based on detecting changes in the headspace gas composition that result from gas ingress through a leak into a sealed container. To detect a gas ingress, the sample is placed in a chamber filled with a tracer gas (CO₂ in case of the CO₂ headspace analysis (ingress method)) or another gas (e.g., N₂ in the case of O₂ headspace analysis (depletion method). A leak defective container will naturally ingress air and an increase of headspace concentration of the tracer gas, respectively the decrease of headspace concentration other gases, which will imply loss of CCI.

The headspace gas analyzers employ tunable diode laser absorption spectroscopy (TDLAS) and incorporate a high sensitivity signal processing technique that is known as frequency modulation spectroscopy (FMS) to provide gas analysis of the headspace within sealed containers. This optical technique can measure a number of physical parameters within the headspace of a container, including specific gas number density and total headspace pressure.

In TDLAS applications, light from a near-infrared (NIR) semiconductor laser is tuned to match the internal vibrational frequency of a target molecule (e.g., tracer gas). When the NIR laser light encounters the target molecules in the sample headspace, any absorption by the targets modulates the amplitude of the frequency of the laser light exiting the sample. A photodetector detects this signal that is subsequently processed by an electrical mixer to generate the FMS absorption signal associated with the target molecules in the sample headspace. The FMS technique increases the detection sensitivity by ~10,000-fold and compensates for both the relatively weak absorption strengths of near-infrared transitions of the target molecules and the relatively short path lengths associated with the container headspace. It is to be noted that this FMS signal processing transforms the original absorption peak into its first derivative. The area and width characteristics of the FMS absorption signal provide information on the target molecule number density and/or pressure.

### Headspace Carbon Dioxide Measurement Principles

The FMS-CO₂ Headspace Carbon Dioxide Analyzer operates on the principles of frequency modulation spectroscopy (FMS) employing a diode laser tuned to match a specific transition energy of the carbon dioxide molecule. During a measurement, the laser frequency is repeatedly scanned over the absorption feature and successive scans are averaged to improve the signal to noise ratio. The averaged intensity of the FMS absorption signal is proportional to the headspace carbon dioxide number density. Standards were filled with certified gas mixtures containing the stated amount of carbon dioxide were used for calibration.

The sealed samples are measured before testing and then stored in the -80 °C freezer containing dry ice for a predefined period of time, and subsequently, the samples are let to thaw for at least 20 minutes prior to the measurement. The CO₂ amount is measured again by a FM-TDLAS. An increase in CO₂ concentration is indicative of a loss of CCI in the freezer.

### Headspace Oxygen Measurement Principles

The FMS-Oxygen Headspace Analyzer operates on the principles of frequency modulation spectroscopy (FMS) employing a diode laser tuned to match a specific transition energy of the oxygen molecule. During a measurement, the laser frequency is repeatedly scanned over the absorption feature and successive scans are averaged to improve the signal to noise ratio. Because of the relatively weak absorption cross-section associated with the oxygen transition, an additional low band-pass filter is used that distorts the canonical FMS absorption signal. Despite this distortion, the averaged intensity of the FMS absorption signal is correlated to the headspace oxygen number density. FMS spectra from oxygen absorption in standards filled with certified gas mixtures of oxygen in nitrogen at ~1 atm total pressure are used for calibration.

The CCI test method relays in the advantage that the controlled rate freezer to be used, utilizes liquid nitrogen to freeze. This generates a highly enriched nitrogen environment inside the freezer. When a sample contains a defect/leak, the initial headspace air (~20 % oxygen) in the test sample will be replaced by nitrogen. Thus, the comparison of headspace oxygen analysis between before and after storage would reveal oxygen depletion, indicating a loss of CCI.

### Examples

### Coating compositions

Sets of glass syringes (Schott syriQ 1.0 ml long, Fiolax clear) were coated with coating compositions. The following coating compositions were used.

| **Component** | **Function** | **Composition A** | **Composition B** |
|---|---|---|---|
| vinyl-terminated polydimethylsiloxane | cross-linkable polysiloxane structural units | 9.59 wt.-% | 10.59 wt.-% |
| methylhydrosiloxane/dimethylsiloxane copolymer | crosslinking polysiloxane | 0.24 wt.-% | 0.26 wt.-% |
| Pt complex | catalyst | 0.13 wt.-% | 0.15 wt.-% |
| silicone oil 20,000 cSt | non-cross-linkable polysiloxane structural units | 3.84 wt.-% | 4.24 wt.-% |
| silicone oil 1,000 cSt | non-cross-linkable polysiloxane structural units | 9.46 wt.-% | 0.00 wt.-% |
| HMDSO | diluent | ad 100 wt.-% | ad 100 wt.-% |

Glass syringes were coated using spray coating. Afterwards, the coating was cured at elevated temperatures in an oven. During curing, the diluent evaporated and the cross-linkable polysiloxane formed a network in a hydrosilylation reaction.

### Coating thickness

Coatings of various thicknesses were applied to glass syringes and cured at 70 °C curing temperature for 60 seconds. The coating was applied to the inner surface of the syringe barrel and the nestling surface at the tip of the syringe. Then, both open ends were closed. One open end was closed using Datwyler FM257 stopper, the other open end was closed using a West W7025 tip cap. The samples were tested in an ethanol-modified dye ingress container closure integrity test at -80 °C as described above. Samples F and G are a state-of-the art silicone oils (DuPont Liveo^{®} D360) with a viscosity of 1,000 cSt and 12,500 cSt, respectively.

| | **Sample A** | **Sample B** | **Sample C** | **Sample D** | **Sample E** | **Sample F** | **Sample G** |
|---|---|---|---|---|---|---|---|
| coating thickness | 1000 nm | 800 nm | 500 nm | 400 nm | 200 nm | 800 nm | 800 nm |
| CCI test | passed | passed | passed | failed | failed | failed | failed |

### Curing temperature

Coatings of compositions A (samples H and I) and B (samples J and K) were cured at different curing temperatures for 60 seconds and tested for standardized break loose and glide force. Coating thickness was 800 nm.

| | **Sample H** | **Sample I** | **Sample J** | **Sample K** |
|---|---|---|---|---|
| Curing temperature | 40 °C | 70 °C | 40 °C | 70 °C |
| BLF | 7.4 ± 0.29 N | 7.6 ± 0.21 N | 7.4 ± 0.32 N | 4.9 ± 0.41 N |
| GF | 1.0 ± 0.08 N | 1.0 ± 0.03 N | 3.5 ± 0.79 N | 2.8 ± 0.33 N |

### DSC measurements

Differential scanning calorimetry was performed on samples of the coatings after curing and scraping of the cured coating from the glass (in accordance with DIN 51007:2019). The experiments were run on a DSC Q2000 (TA Instruments) at a temperature ramp of 10 °C/min in a range of -120 °C to -60 °C. Measurements were confirmed by two repetitions.

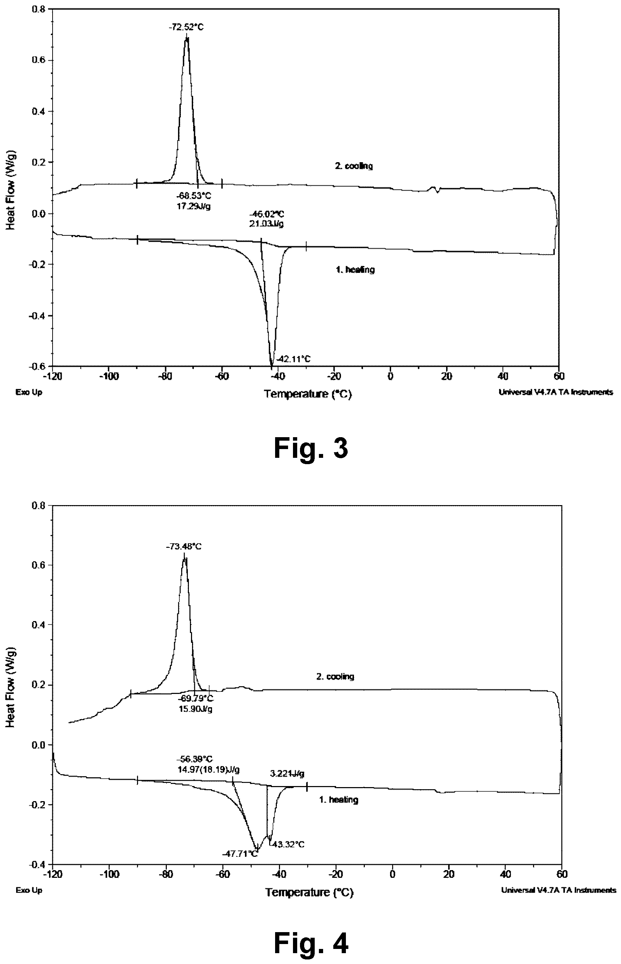

The results for composition A are shown in **Figure 3****.** The exothermal crystallization peak is at -72.52 °C. It extends from -90 °C to -60 °C. The endothermal melting peak is at -42.11 °C and extends from -90 °C to -30 °C. There is an overlap between the two peaks in a temperature range that includes -80 °C.

The results for composition B are shown in **Figure 4****.** The exothermal crystallization peak is at -73.48 °C. It extends from -93 °C to -65 °C. An endothermal melting peak extends from -90 °C to -50 °C and beyond. There is an overlap between the two peaks in a temperature range that includes -80 °C.

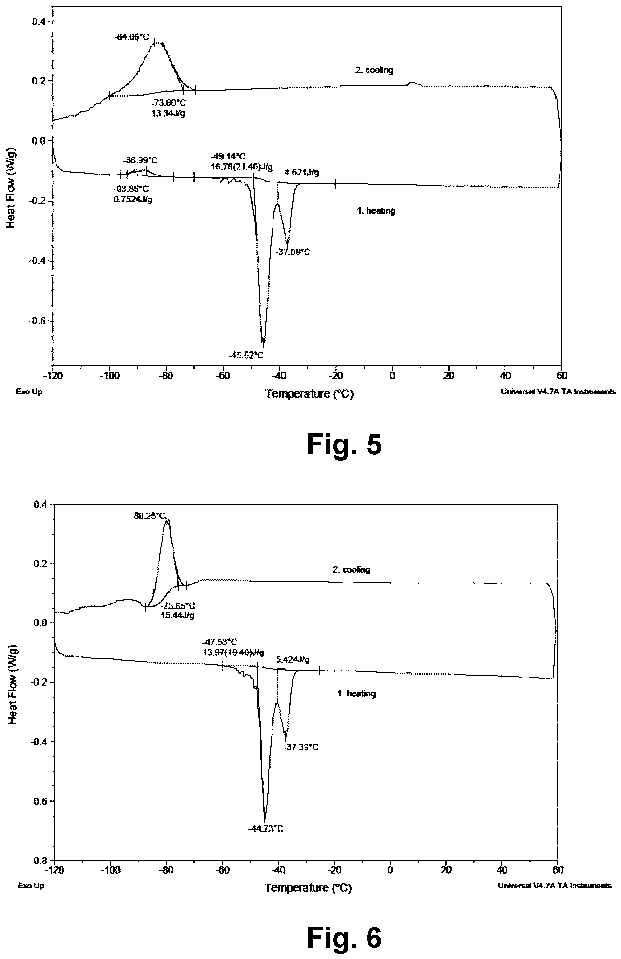

The results for a state-of-the art silicone oil with a viscosity of 1,000 cSt are shown in **Figure 5****.** The exothermal crystallization peak is at -84.06 °C. It extends from -100 °C to -70 °C. An endothermal melting peak extends from -70 °C to -50 °C and beyond. There is no overlap between the two peaks in a temperature range that includes -80 °C.

The results for a state-of-the art silicone oil with a viscosity of 12,500 cSt are shown in **Figure 6****.** The exothermal crystallization peak is at -80.25 °C. It extends from -88 °C to -73 °C. An endothermal melting peak extends from -60 °C to -40 °C and beyond. There is no overlap between the two peaks in a temperature range that includes -80 °C.

### Glass transition temperatures

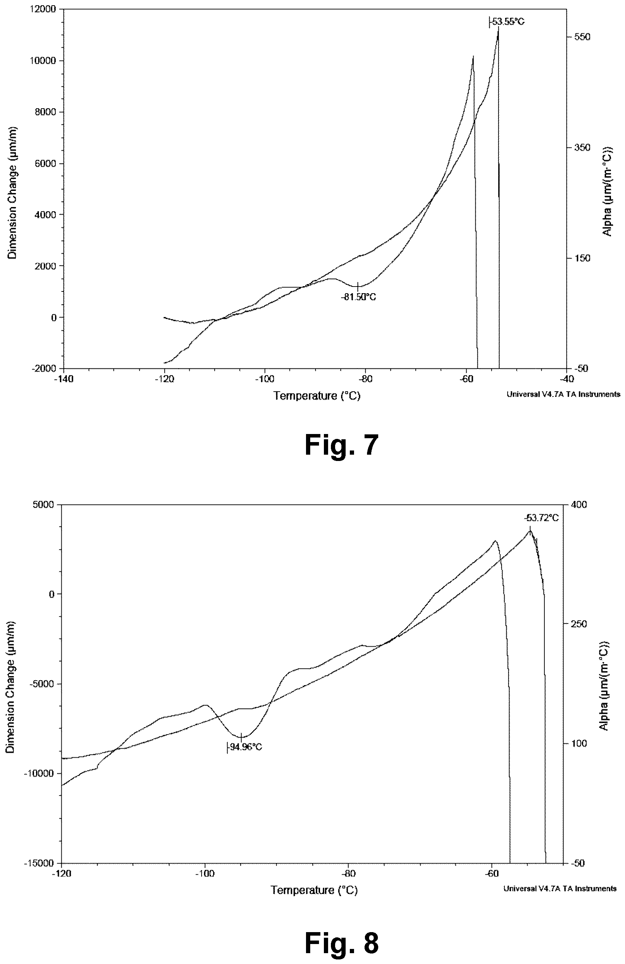

To determine the glass transition temperatures, thermomechanical analysis was performed in expansion mode, using a Q400 thermomechanical analyzer by TA Instruments. The sample was prepared in the same way as for the DSC measurements. Composition A had a glass transition temperature of -81.50 °C, and composition B of -94.96 °C. The results are shown in **Figures 7 and 8****,** respectively.

### Container closure integrity

A plunger side CCI test using CO₂ headspace analysis has been performed on glass syringes coated as described above with composition A and B and using a Datwyler FM257 stopper. The cooling rate has been ca. 1 °C/min. The headspace in the water filled samples has been 4 mm wherein the position of the headspace has been close to the stopper for the vertically oriented samples.

| **Composition** | **Filling medium** | **Orientation** | **Pass* (168 h, -80 °C)** | **Pass* (1 month, -80 °C)** |
|---|---|---|---|---|
| A 1,000 nm | water | vertical | 30/30 | 30/30 |
| | water | horizontal | 30/30 | 30/30 |
| | empty | n/a | 30/30 | 30/30 |
| B 1,400 nm | water | vertical | 30/30 | 30/30 |
| | water | horizontal | 30/30 | 30/30 |
| | empty | n/a | 30/30 | 30/30 |

| | | | | |
|---|---|---|---|---|
| * Leakage values below 10 mbar CO₂ considered as pass. | | | | |

As can be seen, when the syringes are stored filled, unfilled, and differently oriented, the stopper/barrel interface meets CCI at -80 °C.

The same test has been repeated with a cooling rate of 2 °C to 4 °C between room temperature and -80 °C.

| **Composition** | **Filling medium** | **Orientation** | **Pass (168 h, -80 °C)** |
|---|---|---|---|
| A 1,000 nm | empty | n/a | 25/25 |
| | water | horizontal | 25/25 |
| | water | vertical | 25/25 |
| B 1,400 nm | empty | n/a | 25/25 |
| | water | horizontal | 25/25 |
| | water | vertical | 25/25 |

This comparison again demonstrates that using the cooling rates of this disclosure, both coating compositions achieve full CCI for 168 hours at -80 °C for empty and water-filled syringes in horizontal as well as vertical orientation.

For a further test of the effect of the cooling rate on the CCI at -80 °C and -96 °C, four sets of syringes have been coated with composition B in the same manner as described above. The thickness of the coating has been 1,400 nm. Again, the standard Datwyler FM257 stopper has been used for all syringes. The measurements have been made using the CCI test with O₂ headspace analysis over a time of 24 hours at -80 °C and one hour at -96 °C, respectively.

All samples have been filled with 0.7 ml water and dispersed in vertical orientation for sets A-C in a plastic tub with the stopper being on the top side. The samples of set D have been horizontally oriented. Additionally, for each run in the freezer three temperature probes have been placed in close proximity to the tested sample set for monitoring the temperatures rates.

The samples, controls, and sensors have been positioned in the plastic tub (or trays) including slots with temperature probes and slots with defect syringe controls. Similar sensors and controls are also used for horizontal samples.

Set A has been measured at -80 °C with a headspace of 4 mm. Four runs of 30 samples, each, have been tested with different cooling rates from -50 °C to -80 °C.

Set B and set C have been measured at -96 °C with a headspace of 4 mm and 8 mm, respectively. The samples have been cooled at a cooling rate of 3 °C/min to 4 °C/min from -50 °C to -80 °C and held at that temperature for 15 min. With this preconditioning, in each set three runs of 30 samples, each, have been tested with different cooling rates from -80 °C to -96 °C.

Set D has been a repetition of the runs with the highest cooling rate of sets B and C, this time with horizontal orientation of the syringes in trays.

| **Set** | **Temperature** | **Headspace** | **Orientation** | **Cooling rate after preconditioning** | **Pass** |
|---|---|---|---|---|---|
| A | -80 °C | 4 mm | vertical | 3 °C/min to 4 °C/min from -50 °C to -80 °C | 30/30 |
| | -80 °C | 4 mm | vertical | 4 °C/min to 6 °C/min from -50 °C to -80 °C | 30/30 |
| | -80 °C | 4 mm | vertical | 9 °C/min to 10 °C/min from -50 °C to -80 °C | 25/30 |
| | -80 °C | 4 mm | vertical | 15 °C/min to 20 °C/min from -50 °C to -80 °C | 17/30 |
| B | -96 °C | 4 mm | vertical | 0.5 °C/min to 1.5 °C/min from -80 °C to -96 °C | 30/30 |
| | -96 °C | 4 mm | vertical | 2 °C/min to 4 °C/min from -80 °C to -96 °C | 30/30 |
| | -96 °C | 4 mm | vertical | 4 °C/min to 5 °C/min from -80 °C to -96 °C | 30/30 |
| C | -96 °C | 8 mm | vertical | 0.5 °C/min to 1.5 °C/min from -80 °C to -96 °C | 30/30 |
| | -96 °C | 8 mm | vertical | 2 °C/min to 4 °C/min from -80 °C to -96 °C | 30/30 |
| | -96 °C | 8 mm | vertical | 4 °C/min to 5 °C/min from -80 °C to -96 °C | 30/30 |
| D | -96 °C | 4 mm | horizontal | 4 °C/min to 5 °C/min from -80 °C to -96 °C | 30/30 |
| | -96 °C | 8 mm | horizontal | 4 °C/min to 5 °C/min from -80 °C to -96 °C | 30/30 |

The results of set A demonstrate that CCI at -80 °C can be reliably achieved with a cooling rate of ≤ 6 °C/min while at higher rates the number of failed samples increases with increasing rates.

Further, the results of sets B, C, and D demonstrate that independent of the orientation of the syringes a cooling rate of ≤ 5.0 °C/min can be successfully applied in the range from -80 °C to -96 °C for headspaces of 4 mm and 8 mm.

### Reference number list

- **1**: glass container
- **3**: syringe
- **5**: glass wall
- **7**: hollow cylindrical body
- **10**: coating
- **12**: stopper
- **13**: push rod
- **15**: flange
- **18**: nestling surface
- **200**: setup
- **201**: container
- **205**: container volume
- **210**: body
- **212-1, 212-2**: open end
- **230-1**: closure device (stopper)
- **230-2**: closure device (cap)
- **270**: immersion arrangement
- **272**: immersion device
- **274**: ambient liquid
- **L**: amount of ambient liquid

## Claims

1. A method for storing a pharmaceutical composition at a temperature of -80 °C or below comprising
- providing a glass container (1) comprising a hollow cylindrical body (7) having at least one open end (212-1, 212-2) and at least one stopper (12) closing the at least one open end (212-1, 212-2), wherein at least a part of an inner surface of the glass container (1) comprises a coating (10), the coating comprising silicon-organic polymers, the coating (10) having a crystallization temperature range and a melting temperature range determined using differential scanning calorimetry at a temperature change rate of 10 °C/min, wherein the crystallization temperature range and the melting temperature range overlap at a temperature of from -75 °C to -100 °C, particularly at -80 °C;
- filling a pharmaceutical composition into the glass container (1); and
- cooling the glass container (1) with a cooling rate of ≤ 6.0 °C/min in a range of from -50 °C to -80 °C and with a cooling rate of ≤ 5.0 °C/min in a range of from -80 °C to -96 °C.

2. The method according to claim 1, wherein the coating (10) composition has the following composition in percent by weight
| | |
|---|---|
| one or more cross-linkable polysiloxane compounds | 3.0 % to 20.0 % |
| one or more non-cross-linkable polysiloxane compounds | 2.0 % to 15.0 % |
| one or more cross-linking polysiloxane compounds | 0.10 % to 1.50 % |
| one or more catalysts | 0.03 % to 0.50 % |
| one or more diluents | 65.0 % to 92.0 % |

3. The method according to claim 1, wherein the coating (10) composition has the following composition in percent by weight
| | |
|---|---|
| one or more cross-linkable polysiloxane compounds | 8.0 to 20.0 % |
| one or more non-cross-linkable polysiloxane compounds | 2.0 to 10.0 % |
| one or more cross-linking polysiloxane compounds | 0.10 to 1.00 % |
| one or more catalysts | 0.03 to 0.50 % |
| one or more diluents | 69.0 to 90.0 % |

4. The method according to claim 1, wherein the coating (10) composition has the following composition in percent by weight
| | |
|---|---|
| a vinyl-functionalized polysiloxane as a cross-linkable polysiloxane compound | 3.0 % to 20.0 % |
| one or more non-cross-linkable polysiloxane compounds | 2.0 % to 15.0 % |
| a copolymer having dimethylsiloxane and methylhydrosiloxane monomer units as a cross-linking polysiloxane compound | 0.10 % to 1.50 % |
| one or more Pt-containing catalysts | 0.03 % to 0.50 % |
| one or more diluents | 65.0 % to 92.0 % |

5. The method according to any of the preceding claims, wherein the coating (10) was cured at a temperature
- of less than 150 °C, or less than 125 °C, and/or
- of 50 °C or more, or 60 °C or more.

6. The method according to any of the preceding claims, wherein the coating (10) has a thickness
- of 400.0 nm or more, or 500.0 nm or more, 600.0 nm or more, or 700.0 nm or more, 800.0 nm or more, or 900.0 nm or more, or 1,000.0 nm or more; and/or
- of 2,000.0 nm or less, or 1,900.0 nm or less, or 1,800.0 nm or less, or 1,700.0 nm or less, or 1,600.0 nm or less, or 1,500.0 nm or less, or 1,400.0 nm or less.

7. The method according to any of the preceding claims, wherein
- the coating (10) has a glass transition temperature at -60 °C or below, or at -70 °C or below, or at -75 °C or below, or at -80 °C or below; and/or
- the stopper (12) has a glass transition temperature at -80 °C or below, or at -85 °C or below, or at -90 °C or below, or at -95 °C or below, or at -100 °C or below, or below the storing temperature.

8. The method according to one of the preceding claims, wherein, when inserted in the hollow cylindrical body (7) of the glass container (1), a stopper compression ratio of the at least one stopper (12) defined as (Dₛ - D_{b}) / Dₛ, with Dₛ = outer diameter of the uncompressed stopper (12) and D_{b} = inner diameter of the hollow cylindrical body (7), is
- 8.0 % or more, or 10.0 % or more, or 12.0 % or more, or 14.0 % or more, or 15.0 % or more; and/or
- 18.0 % or less, or 17.5 % or less, 17.0 % or less, 16.5 % or less, 16.0 % or less.

9. The method according to one of the preceding claims, wherein the glass container (1) has a standard ethanol-modified dye-ingress tested container closure integrity of at least 168 hours at -80 °C and/or an O₂ headspace analysis tested container closure integrity of at least 1 hour at -96 °C.

10. The method according to one of the preceding claims, wherein
- in addition to the pharmaceutical composition a gas is filled into the glass container (1),
- a volume enclosed by the glass container (1) which is occupied by the gas is defined as headspace of the glass container (1).

11. The method according to claim 10, wherein the gas is or comprises one or more of air, CO₂, N₂, Ar, and/or O₂.

12. The method according to claim 10 or 11, wherein the glass container (1) is oriented vertically after filling and before cooling, such that the headspace of the glass container (1) is located adjacent to the stopper (12).

13. The method according to claim 12, wherein the headspace has a cylindrical volume domain portion which volume domain portion has a specific diameter equal to the inside diameter of the hollow cylindrical body (7) and a specific height, which specific height preferably is measured from the central point of the at least one stopper (12) to the surface of the pharmaceutical composition facing the at least one stopper (12), wherein the specific height has a value of
- 2.0 mm or more, or 3.0 mm or more, or 4.0 mm or more, or 5.0 mm or more, or 6.0 mm or more, or 7.0 mm or more, or 8.0 mm or more, or 9.0 mm or more, or 10.0 mm or more, or 11.0 mm or more, or 12.0 mm or more, or 13.0 mm or more, or 14.0 mm or more, or 15.0 mm or more; and/or
- 15.0 mm or less, or 14.0 mm or less, or 13.0 mm or less, or 12.0 mm or less, or 11.0 mm or less, or 10.0 mm or less, or 9.0 mm or less, or 8.0 mm or less, or 7.0 mm or less, or 6.0 mm or less, or 5.0 mm or less, or 4.0 mm or less, or 3.0 mm or less; and/or
- between 2.0 mm and 15.0 mm, or between 2.0 mm and 10.0 mm, or between 3.0 mm and 9.0 mm, or between 3.0 mm and 5.0 mm, such as 4.0 mm, or between 5.0 mm and 7.0 mm, such as 6.0 mm, or between 7.0 mm and 9.0 mm, such as 8.0 mm.

14. The method according to one of the preceding claims, wherein cooling the glass container (1) comprises keeping the temperature constant for at least 1.0 min, or at least 2.0 min, or at least 3.0 min, or at least 4.0 min, or at least 5.0 min in a range of 1.0 °C to 5.0 °C each above the glass transition temperature of the stopper (12) and the coating (10).

15. The method according to one of the preceding claims, wherein the coating (10) comprises one or more cross-linked polysiloxane structural units and one or more non-cross-linked polysiloxane structural units, wherein a ratio of a weight amount of cross-linked polysiloxane structural units to a weight amount of non-cross-linked polysiloxane structural units in the coating (10) is less than 3.00, and optionally at least 0.40.

16. The method according to one of the preceding claims, wherein the coating (10) comprises at least two non-cross-linked polysiloxane structural units that differ in their viscosities.
